**Europäisches Patentamt**

**European Patent Office**

**Office européen des brevets**

(19)

(11) Veröffentlichungsnummer: **0 021 381**
**B1**

(12) **EUROPÄISCHE PATENTSCHRIFT**

(45) Veröffentlichungstag der Patentschrift:
16.03.83

(51) Int. Cl.³: **C 07 C 21/06, C 07 C 17/34**

(21) Anmeldenummer: **80103494.3**

(22) Anmeldetag: **21.06.80**

(54) Verfahren zur Rückgewinnung von Pyrolyseenergie bei der Herstellung von Vinylchlorid durch thermische Spaltung von 1,2-Dichlorethan.

(30) Priorität: **26.06.79 DE 2925720**

(43) Veröffentlichungstag der Anmeldung:
**07.01.81 Patentblatt 81/1**

(45) Bekanntmachung des Hinweises auf die Patenterteilung:
**16.03.83 Patentblatt 83/11**

(84) Benannte Vertragsstaaten:
**BE DE FR GB IT NL**

(56) Entgegenhaltungen:
**EP-A-0 014 920
DE-B-1 240 426
DE-B-2 313 037**

(73) Patentinhaber: **Uhde GmbH,
Deggingstrasse 10 - 12 Postfach 262,
D-4600 Dortmund 1 (DE)**
Patentinhaber: **HOECHST AKTIENGESELLSCHAFT,
Postfach 80 03 20, D-6230 Frankfurt/Main 80 (DE)**

(72) Erfinder: **Link, Gerhard, Dipl.-Ing.,
Jakob-Steffanstrasse 14, D-6500 Mainz (DE)**
Erfinder: **Fröhlich, Walter, Dr. Dipl.-Chem.,
Kampenwandstrasse 11, D-8261 Burgkirchen (DE)**
Erfinder: **Riedl, Josef, Dr. Dipl.-Chem., Kantstrasse 53,
D-8261 Burgkirchen (DE)**
Erfinder: **Krumböck, Reinhard, Dipl.-Ing.,
Lohnerstrasse 40, D-8261 Burgkirchen (DE)**

ACTORUM AG

## Verfahren zur Rückgewinnung von Pyrolyseenergie bei der Herstellung von Vinylchlorid durch thermische Spaltung von 1,2-Dichlorethan

Die Erfindung betrifft ein Verfahren zur Rückgewinnung von Pyrolyseenergie bei der Herstellung von Vinylchlorid durch thermische Spaltung von 1,2-Dichlorethan, anschliessende Kühlung und Rektifikation des Produktgemisches.

Bei der thermischen Spaltung von 1,2-Dichlorethan wird nach bekannten Verfahren das 1,2-Dichlorethan in einem Reaktionsofen indirekt erhitzt und bei Temperaturen im Bereich von 480 bis 560°C zu Vinylchlorid und Chlorwasserstoff gespalten. Die thermische Spaltung verläuft nicht vollständig und führt zu einem Produktgemisch, das neben 1,2-Dichlorethan und den obengenannten Hauptprodukten der Spaltung zahlreiche Nebenprodukte von unterschiedlicher chemischer Struktur enthält. Diese sind zum Beispiel gesättigte und ungesättigte aliphatische Verbindungen, Aromaten und Russ beziehungsweise Koks. Russ- und Koksabscheidungen im Spaltofen führen dazu, dass in Abständen von wenigen Monaten der Betrieb des Ofens für eine Entkokung unterbrochen werden muss. Die Bildung der Nebenprodukte ist in der Technik zum Teil dadurch bedingt, dass es bisher nicht gelungen ist, völlig reines Dichlorethan mit wirtschaftlich vertretbarem Aufwand für die Spaltung bereitzustellen. Eine andere Ursache ist darin zu sehen, dass bei der vorgegebenen hohen Temperatur die Spaltprodukte thermisch nicht stabil sind und in einer Folge weiterer Reaktionen schliesslich zu Kohlenstoff zerfallen.

Wie in der einschlägigen Patentliteratur beschrieben, können anschliessend die heissen Spaltgase indirekt mit einem Kühlmedium oder direkt mit vorgekühltem Spaltprodukt gekühlt werden.

In der Praxis erwies sich die indirekte Kühlung allerdings nicht als vorteilhaft, da es innerhalb kurzer Zeit zu unerwünschten, betriebsstörenden Koksablagerungen kam.

So wird bei einer konventionell ausgelegten Abkühlung der heissen Spaltprodukte von 540°C auf 200°C bereits nach wenigen Wochen Laufzeit ein störender Druckverlust durch zunehmenden Koksbelag in den Rohren festgestellt.

Nach EP-A-0 014 920, Anmeldenummer 80 100 651.1 ist ein Verfahren zur Rückgewinnung von Pyrolyseenergie bei der Herstellung von Vinylchlorid bekannt, wonach mittels der Pyrolyseenergie der heissen Spaltgase der Sumpf einer Destillationszone indirekt aufgeheizt wird. Die heissen Spaltgase durchströmen dabei den oder die Wärmetauscher produktseitig.

Man war deshalb bisher in der Technik bemüht, den thermisch instabilen Zustand des den Spaltofen verlassenden heissen Gasgemisches so rasch wie möglich zu beenden. Dies wurde erreicht durch eine direkte Kühlung, indem flüssiges, kaltes Dichlorethan in den heissen Gasstrom kontinuierlich eingesprüht wird. Die Spaltgase werden dabei auf eine Temperatur von ca. 130 bis 60°C abgeschreckt. In diesem Temperaturbereich treten im allgemeinen keine Sekundärreaktionen, die zur Verkokung führen, mehr auf. Durch diese Abkühlung werden die Spaltgase teilweise kondensiert. Auf dem Spaltofen mit dem Gasstrom ausgetragene Russpartikel sind dabei in dem flüssigen, gekühlten Produkt suspendiert und können durch Filtereinrichtungen entfernt werden. Zur weiteren Aufarbeitung des Spaltproduktgemisches werden nun entsprechend ihren Siedepunkten der Reihe nach Chlorwasserstoff, Vinylchlorid und schliesslich nicht-umgesetztes 1,2-Dichlorethan von den jeweils höhersiedenden Substanzen abgetrennt und das 1,2-Dichlorethan erneut dem Spaltofen zugeführt.

Nach einer Veröffentlichung in der Zeitschrift «Hydrocarbon Processing, November 1975, Seiten 214/215» wird in den kommerziellen Verfahren der beiden wichtigsten Lizenzgeber in einer Vielzahl von gebauten Anlagen nur die direkte Abkühlung der heissen Produktgase mittels flüssigem, gekühltem Produkt vorgenommen.

An dem bisher seit über 20 Jahren ausgeübten direkten Kühlverfahren ist nachteilig der hohe energetische Aufwand zum Umpumpen der Spaltprodukt-Kreislaufmenge und der vollständige Verlust der im Spaltreaktionsofen aufgewendeten beträchtlichen Wärmemenge.

Es ist ferner bekannt, die aus dem Spaltreaktionsofen austretenden Gase in einem Zyklon von abgeschiedenem Russ zu trennen und anschliessend einer Luftkühlung zu unterwerfen, doch benötigt dieses Verfahren gegenüber der Kühlung mit flüssigem 1,2-Dichlorethan bei hohen Durchsätzen einen erheblich vergrösserten apparativen Aufwand, ohne dass damit die nachteiligen Wärmeverluste behoben würden.

Ausserdem ist die Abkühlung der Spaltgase mit Wasser in einer Stufe beschrieben. Dieses Verfahren ist wegen des möglichst niedrigen Temperaturniveaus, auf das die Gase unter Kondensation vor Einspeisung in die erste, zur Abtrennung des Chlorwasserstoffs dienende Kolonne abgekühlt werden müssen, zur Nutzung des Wärmeinhalts der Spaltgase wenig geeignet. Die Beschreibung enthält überdies keine Anhaltspunkte über die erreichte Abkühlgeschwindigkeit der Spaltgase und über die Laufzeit der eingesetzten Kühler.

Ein weiterer Nachteil der direkten Kühlung ist der grosse apparative und maschinelle Aufwand. So ist für die Abkühlung der Spaltgase von ca. 530°C auf ca. 80°C eine Kreislaufkühlmenge erforderlich, die ca. das 17fache der flüssigen Produktmenge beträgt. Der Wärmetausch des Kreislaufs, das heisst die Abfuhr der Spaltwärme erfolgt bei ca. 80 bis 40°C auf der Seite des flüssigen Spaltproduktes, und bei ca. 25 bis 35°C auf der Kühlwasserseite. Die Kühlung der flüssigen Kreislaufmenge muss unter allen Umständen aufrechterhalten und sichergestellt werden, da es sonst im Quenchturm zu gefährlichen Temperaturanstiegen kommt. Die Sicherheitseinrichtung hierfür besteht einmal aus einer Temperaturverriegelung für den Spaltofen, ausgelöst durch eine Temperaturmessung am Ausgang des Quenchturmes und zum anderen aus einem Notstromaggregat, beziehungsweise Dampfturbinenantrieb für mindestens eine Pumpe der Kreislaufflüssigkeit. Nur auf diese Weise kann die im Spaltofen in der Ausmauerung gespeicherte Hitze bei Abschaltung der Anlage gefahrlos über noch verdampfendes 1,2-Dichlorethan abgeleitet werden.

Der Spaltofen befindet sich in der Regel dicht neben dem Quenchturm. So hat die Verbindungsleitung zum Beispiel nur eine kleine Länge von 1 bis 2 m. Ein Einströmen von 530°C heissen Spaltgasen in einen Quenchturm, der im Sumpf flüssiges Produkt enthält und bei dem nicht sichergestellt ist, dass von oben flüssiges, kaltes Kreislaufprodukt eingesprüht wird, führt zu Materialüberbeanspruchung. Im ungünstigsten Fall kann aus dem Quenchsystem durch Leckagen explosives Medium in unmittelbarer Nähe der Spaltofenbrenner ausströmen.

Der Erfindung liegt die Aufgabe zugrunde, die Nachteile der beschriebenen Verfahren zu beseitigen.

Diese Aufgabe wird erfindungsgemäss gelöst durch ein Verfahren zur Herstellung von Vinylchlorid durch thermische Spaltung von 1,2-Dichlorethan, wobei das Spaltgasgemisch, das den Reaktionsofen verlässt, indirekt mit einem flüssigen oder gasförmigen Temperiermedium gekühlt und anschliessend destillativ aufgetrennt wird, dadurch gekennzeichnet, dass die gesamte Kühlung in mindestens zwei Stufen durchgeführt wird, wobei das Spaltgasgemisch mit einer durchschnittlichen Abkühlgeschwindigkeit von mindestens 1/10 der Temperatur in °C, mit der dieses Gemisch in die indirekte Kühlung eintritt, pro Sekunde bis zum Erreichen einer Temperatur von 220 bis 120°C gekühlt, die dabei an das Temperiermedium abgegebene Wärme zur Heizung von Vorrichtungen verwendet und das Spaltgasgemisch gegebenenfalls zusammen mit dem daraus gebildeten flüssigen Kondensat in mindestens einer anschliessenden Stufe direkt oder indirekt weiter abgekühlt wird.

Unter «Temperiermedium» ist ein Stoff oder Stoffgemisch gemeint, der beziehungsweise das zur Wärmeaufnahme und -abgabe dient, und bequem vom Ort der Wärmeaufnahme zum Ort der Wärmeabgabe geleitet werden kann.

Für die indirekte Kühlung wird vorzugsweise ein Wärmetauscher benutzt, in dem das zu kühlende Spaltgas nur ein einzelnes Rohr durchströmt, das vom Temperiermedium umgeben ist. So wird gewährleistet, dass im Produktstrom keine Zonen oder Wirbelräume vorhanden sind, die eine geringere als die beanspruchte Abkühlgeschwindigkeit aufweisen. Wärmetauscher anderer Konstruktion, wie zum Beispiel Plattenwärmetauscher oder Röhrenwärmetauscher, wobei das Temperiermedium in den Röhren und das Spaltgas ausserhalb der Röhren strömen kann, wie auch umgekehrt, eignen sich.

Die direkte Abkühlung des Spaltgases kann vorzugsweise durch Inkontaktbringen mit einer kalten Flüssigkeit, die mindestens 50 Gew.-% 1,2-Dichlorethan enthält, erfolgen. Dies kann beispielsweise durch Versprühen der Flüssigkeit im Gasstrom oder vermittels Durchleiten von Spaltgas und Flüssigkeit im Gegenstrom in einer Kolonne geschehen. Insbesondere wird gereinigtes, dem Prozess zurückgeführtes 1,2-Dichlorethan eingesetzt.

Die durchschnittliche Abkühlgeschwindigkeit während der indirekten Kühlung der heissen Spaltgase auf 220 bis 120°C soll mindestens je Sekunde 1/10 der Temperatur (nachfolgend TA genannt; gemessen in °C) betragen, mit der die Spaltgase in die Zone der indirekten Kühlung eintreten. Sie wird durch Messung der Temperaturdifferenz zwischen dem Eintritt des Spaltgases in die Kühlzone und dem Gasaustritt unter Berücksichtigung der mittleren Verweilzeit des Spaltgases in der Kühlzone ermittelt. Bei Abkühlgeschwindigkeiten unter TA/10 s tritt infolge Nebenreaktionen eine zu starke Verunreinigung des Spaltgasgemisches mit Nebenprodukten ein. Nach oben ist die Abkühlgeschwindigkeit durch die apparativen Ausgestaltungsmöglichkeiten des Kühlers sowie durch wirtschaftliche Erwägungen begrenzt, im allgemeinen werden durchschnittliche Abkühlgeschwindigkeiten von je Sekunde 1/2 der Eingangstemperatur TA nicht überschritten. Vorzugsweise wird eine durchschnittliche Abkühlgeschwindigkeit des Spaltgasgemisches von je Sekunde 1/4 bis 1/9 der Eingangstemperatur TA des Spaltgasgemisches und insbesondere eine solche von je Sekunde 1/5 bis 1/7 dieser Eingangstemperatur TA eingehalten.

Bei der üblichen thermischen Spaltung von 1,2-Dichlorethan verlässt das Spaltgasgemisch den Reaktionsofen unter einem Druck von etwa 1,5 bis 2,5 MPa mit einer Temperatur von etwa 560 bis 480°C. Es wird mit einem flüssigen oder gasförmigen Temperiermedium bis zum Erreichen einer Temperatur von 220 bis 120°C, vorteilhaft von 200 bis 150°C, indirekt gekühlt. Eine Abkühlung auf Werte oberhalb 220°C ist zwar prinzipiell möglich, doch verschlechtert sie die Wärmeausnutzung und ist daher aus wirtschaftlichen Gründen weniger interessant. Bei einer Abkühlung auf unter 120°C wird das Temperiermedium nicht mehr ausreichend erwärmt, um eine gute und vielseitige Wärmeausnutzung zu gewährleisten.

Vorzugsweise wird ein bei 100°C flüssiges Temperiermedium eingesetzt, dessen Dampfdruck bei 220°C 2,5 MPa nicht übersteigt. Solche Temperiermedien sind beispielsweise schwerflüchtige Mineralöle, Diphenyl, Silikonöle.

In einer weiteren, bevorzugten Ausführungsform des vorliegenden Verfahrens wird zur indirekten Kühlung ein Temperiermedium eingesetzt, dessen Siedepunkt bei einem Druck von 98,1 kPa zwischen 70 und 110°C liegt, beispielsweise Wasser, 1,2-Dichlorethan oder flüssige Gemische, die mindestens 50 Gew.-% 1,2-Dichlorethan enthalten.

Der Rest-Prozentgehalt des Gemisches soll aus Stoffen bestehen, die das Material des Wärmetauschers möglichst nicht angreifen. Sofern Chlor oder chlorwasserstoffhaltiges 1,2-Dichlorethan verwendet wird, muss ein entsprechend beständiges Baumaterial für den Wärmetauscher vorgesehen werden.

In einer weiteren bevorzugten Ausführungsform des erfindungsgemässen Verfahrens wird das Spaltgasgemisch, das den Reaktionsofen mit einer Temperatur von ca. 560 bis 480°C verlässt, zunächst durch Inkontaktbringen mit einem flüssigen, mindestens 50 Gew.-% 1,2-Dichlorethan enthaltenden Produkt direkt und erst danach mit einem flüssigen oder gasförmigen Temperiermedium indirekt gekühlt, wobei die indirekte Kühlung erst einsetzt, wenn das Spaltgasgemisch eine Temperatur von etwa 430 bis 350°C, vorzugsweise von etwa 400 bis 370°C, erreicht hat, und bis zum Erreichen einer Temperatur von 220 bis 120°C, vorzugsweise von 200 bis 150°C, weitergeführt wird, wobei ferner die in das Temperiermedium abgegebene Wärme zur Heizung von Vorrichtungen verwendet und das Spaltgasgemisch gegebenenfalls zusammen mit dem daraus gebildeten Kondensat in mindestens einer anschliessenden Stufe direkt oder indirekt weiter abgekühlt wird.

Das Inkontaktbringen erfolgt zweckmässig durch Versprühen oder sonstige feine Verteilung wie beispielsweise Ausbreiten in einer dünnen Schicht des flüssigen 1,2-Dichlorethan enthaltenden Produktes in einem von dem heissen Spaltgasgemisch durchströmten Raum.

Das flüssige 1,2-Dichlorethan kann bis zu 50 Gew.-% andere Stoffe enthalten, die mit dem heissen Spaltgasgemisch nicht oder nur in untergeordnetem Mass chemisch reagieren. Vorzugsweise wird ein 1,2-Dichlorethan verwendet, das aus der Direktchlorierung und/oder aus der Oxychlorierung von Ethylen stammt und/oder das aus dem thermischen Spaltprozess zur Gewinnung von Vinylchlorid durch Kühlung der Spaltgase gewonnen wurde und die bei diesen Prozessen üblichen bekannten Begleitstoffe enthält.

Bei der indirekten Kühlung auf 220 bis 120°C werden dieselben durchschnittlichen Abkühlgeschwindigkeiten verwendet, wie zuvor beschrieben. Bei relativ niedrigen Eingangstemperaturen TA des Spaltgasgemisches in die Zone der indirekten Kühlung können im allgemeinen auch noch etwas höhere durchschnittliche Abkühlgeschwindigkeiten angewendet werden, als sich aus den weiter oben gemachten Ausführungen über den Vorzugsbereich von TA/4 bis TA/9 ergibt. Beispielsweise werden bei TA = 360°C auch noch bei Abkühlgeschwindigkeiten von 100 bis 120°C/s = TA/3,2 s bis TA/2,66 s gute Ergebnisse mit nicht allzu hohem apparativem Aufwand erzielt.

Für die Ausführungsform des erfindungsgemässen Verfahrens mit Vorkühlung des heissen Spaltgasgemisches mit 1,2-Dichlorethan werden für die anschliessende indirekte Kühlung vorteilhaft dieselben Temperiermedien eingesetzt wie bei dem Verfahren ohne Vorkühlung mit 1,2-Dichlorethan.

In einer anderen bevorzugten Ausführungsform des Verfahrens wird das Spaltgasgemisch in folgenden Stufen gekühlt:

In einer ersten Stufe durch indirekte Kühlung von der Spaltofen-Ausgangstemperatur auf etwa 220°C, dann in einer zweiten Stufe unter absatzweiser Zuführung einer im wesentlichen 1,2-Dichlorethan enthaltenden Flüssigkeit durch teilweise direkte, teilweise indirekte Kühlung auf etwa 140°C und anschliessend in mindestens einer weiteren Stufe durch direkte oder indirekte Kühlung auf die Eingangstemperatur zu der Kolonne, in der Chlorwasserstoff aus den Produkten der thermischen Spaltung von 1,2-Dichlorethan abgetrennt wird.

Das zuletzt beschriebene, bevorzugte Verfahren wird nachfolgend bis einschliesslich Seite 13, Absatz 3 (Zeile 24) näher erläutert.

Die Kühlung in der ersten und zweiten Stufe wird wie bei den anderen Ausführungsformen des erfindungsgemässen Verfahrens vorzugsweise in einem Wärmetauscher vorgenommen, in dem das zu kühlende Spaltgas nur ein einzelnes Rohr durchströmt, das vom Temperiermedium umgeben ist. Wärmetauscher anderer Konstruktion, wie zum Beispiel Platten-Wärmetauscher oder Röhren-Wärmetauscher, bei denen das Temperiermedium innerhalb der Röhren oder Platten und das Spaltgas ausserhalb der Röhren oder Platten strömen kann wie auch umgekehrt, eignen sich ebenfalls. Sowohl die beiden Einrohr-Wärmetauscher wie auch die genannten anderen Konstruktionen können in Strömungsrichtung der zu kühlenden Spaltgase einen sich kontinuierlich oder absatzweise verengenden Querschnitt aufweisen, um die durch die Abkühlung erzeugte Volumenverminderung der Gase ganz oder teilweise zu kompensieren und damit die Strömungsgeschwindigkeit gleichmässiger zu halten als dies bei konstantem Durchsatzquerschnitt gegeben wäre.

Die durchschnittliche Abkühlgeschwindigkeit der Spaltgase in der ersten Stufe soll mindestens je Sekunde 1/10 und in der zweiten Stufe mindestens je Sekunde 1/5 der Temperatur betragen, mit der die Spaltgase in die jeweilige Kühlstufe eintreten. Die durchschnittliche Abkühlgeschwindigkeit wird, wie weiter oben beschrieben, ermittelt. Nach oben ist die Abkühlgeschwindigkeit durch die apparativen Ausgestaltungsmöglichkeiten des Kühlers sowie durch wirtschaftliche Erwägungen begrenzt. Im allgemeinen werden durchschnittliche Abkühlgeschwindigkeiten in der ersten Stufe von je Sekunde 1/2 und in der zweiten Stufe von je Sekunde 1/1 der Eingangstemperatur TA nicht überschritten. Vorzugsweise wird eine durchschnittliche Abkühlgeschwindigkeit des Spaltgasgemisches in der ersten Kühlstufe von je Sekunde 1/4 bis 1/9 und in der zweiten Kühlstufe von je Sekunde 1/2 bis 1/4 der Eingangstemperatur TA des Spaltgasgemisches eingehalten. In der ersten Kühlstufe wird insbesondere mit einer Kühlgeschwindigkeit von 1/5 bis 1/7 der Eingangstemperatur in dieser Stufe gearbeitet.

In der ersten Stufe wird das Spaltgasgemisch von der Spaltofen-Ausgangstemperatur, im allgemeinen von ca. 560 bis 480°C, auf eine Temperatur von 250 bis 170°C, vorzugsweise auf etwa 220°C, abgekühlt, in der zweiten Stufe erfolgt die weitere Abkühlung des Spaltgasgemisches von der Ausgangstemperatur der ersten Stufe auf etwa 150 bis 110°C, vorzugsweise etwa 140°C.

Zwischen den beiden Kühlstufen, die zweckmässig in zwei getrennten Apparaten vorgenommen werden, jedoch auch in einem gemeinsamen Einrohrkühler durchgeführt werden können, ist eine Eingabevorrichtung für eine Flüssigkeit, die im wesentlichen 1,2-Dichlorethan enthält, vorgesehen. Diese Zugabevorrichtung kann ein einfaches Rohr sein, vorzugsweise trägt dieses Rohr an seinem Ende innerhalb des Rohres, in dem Spaltgasgemisch geführt wird, eine Verteilervorrichtung für die eingeführte Flüssigkeit, beispielsweise eine Düse oder eine mit Löchern versehene Platte beziehungsweise Kugel.

Die Einführungsleitung für die 1,2-dichlorethanhaltige Flüssigkeit enthält zweckmässig ausserhalb des Kühlers für die Spaltgase eine Fördervorrichtung, beispielsweise eine Pumpe, die es ermöglicht, die Flüssigkeit kurzzeitig in relativ grossen Mengen in den das Spaltgasgemisch führenden Bereich des Wärmetauschers der zweiten Stufe gegen den dort herrschenden Druck einzuspeisen. Ausserdem ist zweckmässig in der das Spaltgasgemisch führenden Leitung zwischen der ersten und zweiten Kühlstufe eine Vorrichtung vorgesehen, beispielsweise ein druckgeregeltes Ventil, die den Druck in der ersten Kühlstufe weitgehend konstant hält, bei einem plötzlichen Druckabfall in der zweiten Kühlstufe.

Während des Betriebes der beiden Kühlstufen wird in Abständen von 20 bis etwa 500 Stunden, vorzugsweise in Abständen von 50 bis etwa 200 Stunden, eine im wesentlichen 1,2-Dichlorethan enthaltende Flüssigkeit in den das Spaltgasgemisch führenden Raum zwischen den beiden Kühlstufen eingespeist. Die Menge der eingespeisten Flüssigkeit beträgt von 1,5 bis 5,0 kg/min je kg/min des die Kühlzonen durchströmenden Spaltgasgemisches.

Durch die absatzweise Eingabe der im wesentlichen 1,2-Dichlorethan enthaltenden Flüssigkeit wird die Laufzeit des gesamten Kühlsystems verlängert und der Wärmeübergang in der zweiten Kühlstufe verbessert, da hierdurch eine Belagsbildung von aus dem Spaltgas gebildeten Produkten an den Wandungen des Spaltgas führenden Raumes in der zweiten Kühlstufe vermieden oder doch weitgehend verringert wird. Wird die Flüssigkeit in Zeitabständen von wesentlich über 500 Stunden eingeführt und während einer Zeit von unter 1 Minute, so wird der erstrebte Effekt nicht oder nur unvollkommen erreicht. Das gleiche gilt, wenn die Menge der eingespeisten Flüssigkeit unter 1,5 kg/min je kg/min Spaltgas bleibt. Wird die Einspeisung in Abständen von 120 Stunden und/oder während einer Zeitdauer von über 30 Minuten je Einspeisung vorgenommen oder überschreitet die eingespeiste Menge den Wert von 5,0 kg/min je kg/min Spaltgas wesentlich, so ist zwar immer noch ein guter Wärmeübergang vom Spaltgasgemisch auf das Temperiermedium in der zweiten Kühlstufe gewährleistet, doch wird die an das Temperiermedium abgegebene Wärme vergleichsweise klein und damit das Verfahren wenig wirtschaftlich sowie durch die häufigen und/oder übergrossen Eingaben von Flüssigkeit umständlich und aufwendig. Die Menge Spaltgas in kg/min, die die Kühlstufen durchströmt, ist leicht zu bestimmen, da sie praktisch mit der in den Spaltofen eingegebenen Menge 1,2-Dichlorethan gleich ist.

Die im wesentlichen 1,2-Dichlorethan enthaltende Flüssigkeit kann reines 1,2-Dichlorethan sein, kann aber neben diesem noch bis zu 50 Gew.-%, bezogen auf die Flüssigkeit, andere Substanzen enthalten, insbesondere solche, die als übliche Verunreinigungen bei der Herstellung von 1,2-Dichlorethan durch Chlorierung oder Oxychlorierung von Ethylen anfallen oder die nach der thermischen Spaltung im nichtumgesetzten 1,2-Dichlorethan nach Abtrennung des Chlorwasserstoffs und des Vinylchlorids noch enthalten sind. Solche Stoffe sind beispielsweise:

Vinylchlorid; Ethylchlorid; 1,1-Dichlorethylen; 2-Chlorbutadien-(1,3); 1,1-Dichlorethan; Tetrachlorkohlenstoff; 1,1,2-Trichlorethylen; 1,1,2-Trichlor-

ethan; Ethylenchlorhydrin; Chloroform; Benzol und andere. Vorzugsweise wird eine Flüssigkeit eingesetzt, die zu mindestens 60 Gew.-% aus 1,2-Dichlorethan besteht.

Besonders gute Ergebnisse werden erhalten, wenn von der im wesentlichen 1,2-Dichlorethan enthaltenden Flüssigkeit 2,5 bis 3,5 kg/min je kg/min Spaltgas vor der zweiten Kühlstufe in den weiter oben angegebenen Zeiten und Zeitintervallen in den Spaltgasstrom eingespeist werden.

Die im wesentlichen 1,2-Dichlorethan enthaltende Flüssigkeit wird vorteilhaft mit einer Temperatur von 20 bis 70°C in das Spaltgasgemisch eingetragen. Insbesondere kann hierzu ein 1,2-Dichlorethan verwendet werden, das aus der Direktkühlung (Quenche) der Spaltgase von der dritten Kühlstufe stammt, in der die Spaltgase von 150 bis 110°C auf die Eingangstemperatur zu der Kolonne abgekühlt werden, in der Chlorwasserstoff aus den Produkten der thermischen Spaltung von 1,2-Dichlorethan abgetrennt wird, das heisst auf Temperaturen von etwa 0 bis 70°C.

Die Spaltgase, die mit einer Temperatur von 150 bis 110°C die zweite Kühlstufe verlassen, enthalten im allgemeinen bereits kondensierte flüssige Anteile, insbesondere wenn kurz zuvor die weiter oben näher beschriebenen Mengen einer im wesentlichen aus 1,2-Dichlorethan bestehenden Flüssigkeit vor oder während der Kühlung des Spaltgasgemisches in der zweiten Kühlstufe in das Spaltgasgemisch eingetragen werden. Diese Mischung aus flüssigen und gasförmigen Bestandteilen wird nun entweder direkt durch Eintragen von weiterem 1,2-Dichlorethan oder indirekt, beispielsweise durch Kühlung mit Wasser oder flüssigem 1,2-Dichlorethan, auf die Eingangstemperatur zu der Kolonne, in der gasförmiger Chlorwasserstoff abgetrennt wird, vorzugsweise in zwei weiteren Stufen, abgekühlt. Vor Eintritt in diese Kolonne, gegebenenfalls auch schon zwischen der zweiten und dritten Kühlstufe, werden feste Bestandteile aus dem Gas-Flüssigkeitsgemisch durch übliche Methoden, beispielsweise Filtrieren, abgetrennt und aus dem weiteren Prozess ausgeschieden. Die Weiterverarbeitung des 1,2-dichlorethanhaltigen gekühlten Spaltgasgemisches zur Abtrennung des Chlorwasserstoffs des Vinylchlorids sowie weiterer leichter und schwerer als 1,2-Dichlorethan siedender Verbindungen erfolgt nach bekannten Verfahren im allgemeinen durch Destillation, gegebenenfalls unter Chlorzusatz. Das so zurückgewonnene gereinigte 1,2-Dichlorethan wird wie üblich in den Prozess zurückgeführt, das heisst erneuter thermischer Spaltung unterworfen, ein Teil hiervon kann, wie vorstehend beschrieben, als Zusatz-Flüssigkeit für die Spaltgaskühlung verwendet werden.

Die in der ein- oder mehrstufigen indirekten Kühlung auf Spaltgas-Ausgangs-Temperaturen von 220 bis 120°C vom Temperiermedium aufgenommene Wärme kann zur Heizung der verschiedensten Vorrichtungen verwendet werden, beispielsweise von solchen Vorrichtungen, die das 1,2-Dichlorethan vor Eintritt in den Spaltofen aufheizen und gegebenenfalls verdampfen, oder zur Erwärmung von flüssigen Produkten, die den Destillationsapparaten im weiteren Verlauf der destillativen Auftrennung des kondensierten Spaltgasgemisches zugeführt werden. Die vom Temperiermedium bei der indirekten Kühlung aufgenommene Wärme kann aber auch ausserhalb des Prozesses der Herstellung von Vinylchlorid durch thermische Spaltung von 1,2-Dichlorethan verwendet werden, beispielsweise zur Erzeugung von elektrischem Strom, Heizung und Reaktionsgefässen, Rektifizierapparaten oder Gebäuden.

Die zur indirekten Kühlung des Spaltgasgemisches verwendeten Wärmetauscher können zweckmässig mit Einrichtungen versehen werden, die Undichtigkeiten in der Trennwand zwischen Spaltgasgemisch und Temperiermedium anzeigen, um in diesem Fall sofort Massnahmen zur Verhinderung nachhaltiger Betriebsstörungen ergreifen zu können. Zweckmässig wird hierzu ein zweiter Wärmetauscher bereitgehalten, auf den sofort bei Auftreten von Undichtigkeiten oder zur Reinigung des bisher betriebenen Wärmetauschers umgeschaltet werden kann.

Durch das erfindungsgemässe Verfahren wird ein wesentlicher Teil des grossen apparativen und maschinellen Aufwandes einer direkten Abkühlung mit der grossen Kreislauf-Kühlmenge vermieden. Infolge der im indirekten Wärmetauscher vorhandenen Temperiermediumsmenge wird bei Stromausfall in der Anlage die sichere Abführung der Speicherhitze im Spaltofen gewährleistet.

Durch das erfindungsgemässe Verfahren wird darüber hinaus wertvolle Wärmeenergie, die bisher verlorenging, nutzbar gemacht, wodurch die Wirtschaftlichkeit des Herstellverfahrens für Vinylchlorid durch thermische Spaltung von 1,2-Dichlorethan wesentlich verbessert wird. Ferner wird die Abführung von Überschusswärme an Kühlwasser und damit die Umweltbelastung deutlich vermindert.

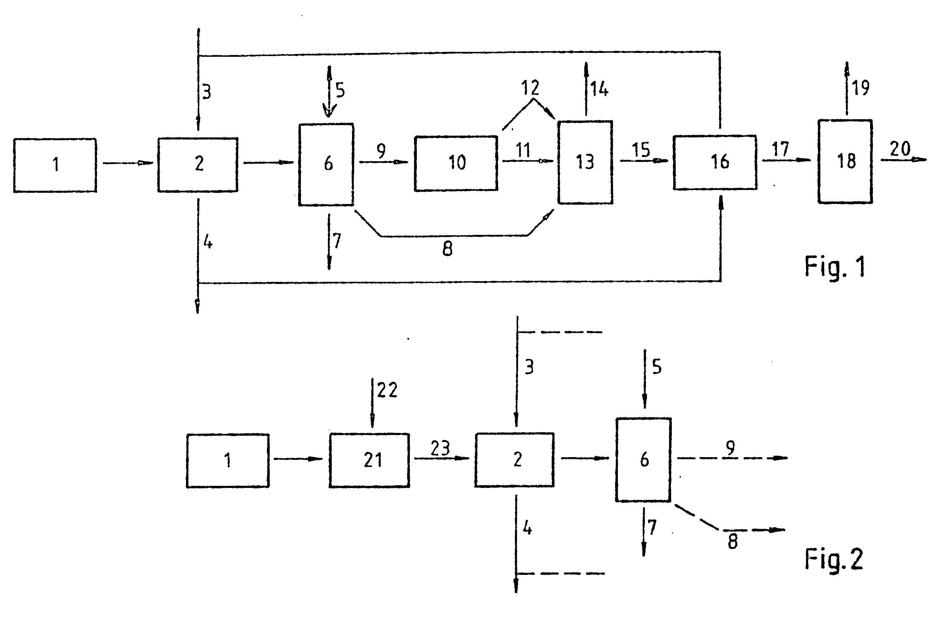

Anhand von schematischen Skizzen wird nachfolgend in den Figuren 1, 2 und 3 der Ablauf des erfindungsgemässen Verfahrens gezeigt und ausserdem in den Beispielen 1 bis 5 näher erläutert. Die Ausführbarkeit des Verfahrens ist nicht auf die gezeigten Beispiele beschränkt. Die Druckangaben in den Beispielen beziehen sich auf absoluten Druck. Zum Vergleich werden zwei Versuche (A und B) mit niedrigen durchschnittlichen Abkühlgeschwindigkeiten beigefügt.

Gemäss dem Fliessschema der Fig. 1 werden die aus dem Spaltofen 1 mit einer Temperatur von 480 bis 560°C austretenden Gase in einem Wärmetauscher 2 mit hoher Geschwindigkeit auf eine Temperatur von 120 bis 220°C abgekühlt, wobei unter einem Druck von 1,4 bis 2,4 MPa eine Teilkondensation der Spaltgase erfolgt. Zur Energieaufnahme aus den Spaltgasen dient ein Temperiermedium, beispielsweise entsalztes Wasser, das über Leitung 3 dem Wärmetauscher 2 zugeführt wird. Die aus den Spaltgasen gewonnene Energie wird mit dem Temperiermedium über die Leitung 4 abgeführt und kann für die Heizung, beispielsweise von Vorrichtungen, die zur Auftrennung der Spaltgase in ihre Komponenten dienen, insbesondere zur Heizung des Produktes, aus dem Vinylchlorid in einer Kolonne abgetrennt wird, verwendet werden. Zur weiteren Abkühlung der Spaltgase auf eine Temperatur von 100 bis 60°C wird das Gas mit 1,2-Dichlorethan von ca. 10 bis 50°C in einer Vorrichtung 6 gewaschen, wo gleichzeitig feine, aus dem Spaltofen mitgerissene Russpartikel aus dem Gasstrom abgetrennt und über die Leitung 7 abgezogen werden. Aus der Waschvorrichtung 6 werden die gasförmigen und flüssigen Reaktionsprodukte getrennt abgeleitet. Die flüssigen, im wesentlichen aus 1,2-Dichlorethan bestehenden Anteile werden über die Leitung 8 direkt der zur Abtrennung des Chlorwasserstoffs dienenden Kolonne 13 zugeführt. Die gasförmigen Anteile werden über Leitung 9 einer Vorrichtung 10 zugeführt, in der sie auf 0 bis 40°C gekühlt und sowohl die hierbei verflüssigten Anteile wie auch die gasförmig gebliebenen über die Leitungen 11 und 12 ebenfalls der Kolonne 13 zugeführt werden. Hier wird der Chlorwasserstoff abgetrennt und über Leitung 14 abgeführt. Das flüssige Sumpfprodukt wird über Leitung 15 einem Wärmetauscher 16 zugeführt, der vorteilhaft mit dem erwärmten Temperiermedium aus Leitung 4 geheizt wird. Das hierbei abgekühlte Medium fliesst zweckmässig zurück in die Leitung 3. Das erwärmte Sumpfprodukt der Kolonne 13 gelangt über Leitung 17 in die Kolonne 18, in der Vinylchlorid abgetrennt und über die Leitung 19 abgeführt wird. Das flüssige Sumpfprodukt der Kolonne 18 wird in bekannter Weise über Leitung 20 mindestens einer Kolonne zugeführt, in der die weitere Auftrennung des Produktgemisches, insbesondere zum Zwecke der Rückgewinnung des 1,2-Dichlorethans erfolgt. Letzteres wird dem Spaltofen 1, gegebenenfalls nach Vorwärmung unter Verwendung des warmen Temperiermediums aus Leitung 4, wieder zugeführt.

Fig. 2 zeigt eine bevorzugte Ausführungsform des erfindungsgemässen Verfahrens.

Die aus dem Spaltofen 1 mit einer Temperatur von 560 bis 480°C austretenden Gase werden einer Vorrichtung 21 zugeführt, in der sie durch Zugabe über Leitung 22 von einer abgemessenen Menge einer Flüssigkeit, die mindestens 50 Gew.-% 1,2-Dichlorethan enthält und eine Temperatur von etwa 10 bis 50°C besitzt, auf 430 bis 350°C abgekühlt werden. Die gekühlten Gase, zusammen mit der verdampften zugeleiteten Flüssigkeit, werden nun über die Leitung 23 dem in Fig. 1 gezeigten Wärmetauscher 2 zugeführt und dann weiter verfahren wie in Fig. 1 beschrieben.

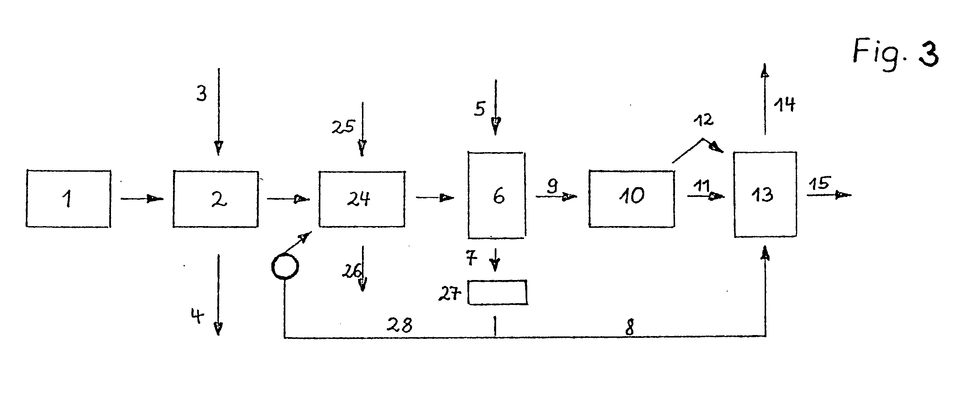

Fig. 3 zeigt eine andere bevorzugte Ausführungsform des erfindungsgemässen Verfahrens.

Die aus dem Spaltofen 1 mit einer Temperatur von 480 bis 560°C austretenden Gase werden in einem Wärmetauscher 2 mit hoher Geschwindigkeit auf eine Temperatur von etwa 220°C abgekühlt und anschliessend einem zweiten Wärmetauscher 24 zugeführt, wo die weitere Abkühlung bis auf etwa 140°C erfolgt, wobei unter einem Druck von 1,4 bis 2,4 MPa eine Teilkondensation der Spaltgase erfolgt. Zur Energieaufnahme aus den Spaltgasen dient ein Temperiermedium, beispielsweise entsalztes Wasser, das über die Leitung 3 und 25 den Wärmeaustauschern 2 und 24 zugeführt wird. Die aus den Spaltgasen gewonnene Energie wird mit dem Temperiermedium über die Leitung 4 beziehungsweise 26 abgeführt und kann für die Heizung, beispielsweise von Vorrichtungen, die zur Auftrennung der Spaltgase in ihre Komponenten dienen, insbesondere zur Heizung des Produktes, aus dem Vinylchlorid in einer Kolonne abgetrennt wird oder des Produktes, aus dem 1,2-Dichlorethan in einer Kolonne abgetrennt wird, verwendet werden. Zur weiteren Abkühlung auf eine Temperatur von 70 bis 60°C wird das Gas mit 1,2-Dichlorethan von ca. 10 bis 50°C, welches über die Leitung 5 zugeführt wird, in einer Vorrichtung 6 gewaschen, wo gleichzeitig feine, aus dem Spaltofen mitgerissene Russpartikel aus dem Gasstrom abgetrennt, über die Leitung 7 abgezogen und über die Vorrichtung 27 von der Flüssigkeit abgetrennt werden. Die so gereinigten flüssigen, im wesentlichen aus 1,2-Dichlorethan bestehenden Anteile werden einesteils über die Leitung 8 direkt der zur Abtrennung des Chlorwasserstoffs dienenden Kolonne 13 zugeführt, andernteils über die Leitung 28 mit Pumpe und Dosiervorrichtung in Zeitintervallen in den zweiten Wärmetauscher 24 eingespeist. Die gasförmigen Anteile aus der Vorrichtung 6 werden über Leitung 9 einer Vorrichtung 10 zugeführt, in der sie auf 0 bis 40°C gekühlt und sowohl die hierbei verflüssigten Anteile wie auch die gasförmig gebliebenen über die Leitungen 11 und 12 ebenfalls der Kolonne 13 zugeführt werden. Hier wird der Chlorwasserstoff abgetrennt und über Leitung 14 abgeführt. Das flüssige Sumpfprodukt wird über Leitung 15 weiteren Vorrichtungen zugeführt, die zur Abtrennung des Vinylchlorids und zur Wiedergewinnung von möglichst reinem nicht-umgesetztem 1,2-Dichlorethan dienen. Letzteres wird dem Spaltofen 1, gegebenenfalls nach Vorwärmung unter Verwendung des warmen Temperiermediums aus Leitung 4, wieder zugeführt (nicht gezeigt).

*Beispiel 1*

Es wird nach Schema-Figur 1 verfahren.

Ein Spaltofen wird stündlich mit 9,98 Gewichtsteilen 1,2-Dichlorethan folgender Zusammensetzung beschickt:

|  | Gew.-% |
|---|---|
| Vinylchlorid | 0,1 |
| Ethylchlorid | 0,004 |
| 1,1-Dichlorethylen | 0,014 |
| 2-Chlorbutadien-(1,3) | 0,034 |
| 1,1-Dichlorethan | 0,052 |
| Tetrachlorkohlenstoff | 0,004 |
| Benzol | 0,159 |
| Chloroform | 0,011 |
| 1,1,2-Trichlorethylen | 0,044 |
| 1,2-Dichlorethan | 99,517 |
| 1,1,2-Trichlorethan | 0,004 |
| Ethylenchlorhydrin | 0,002 |
| Unbekannte | 0,053 |

Bei einem Druck von 2,1 MPa werden stündlich 5,46 Gewichtsteile des 1,2-Dichlorethans gespalten.

Der Umsatz beträgt 55%.

Die den Spaltofen verlassenden 540°C heissen Gase werden durch einen Einröhren-Wärmetauscher mit einem einseitig angeflanschten U-förmigen Rohr geleitet. Als Temperiermedium wird vollentsalztes Wasser verwendet. Die Verweileit der Spaltgase im Wärmetauscher beträgt 4 s. Am Ausgang des U-Rohres werden 200° gemessen. Die durchschnittliche Abkühlgeschwindigkeit der Spaltgase beträgt 85°C/s bei einem Druckverlust der Spaltgase im Wärmetauscher von 0,2 MPa.

Durch indirekten Wärmetausch mit den heissen Spaltgasen werden stündlich 1,65 Gewichtsteile Wasserdampf mit einem Druck von 0,8 MPa erzeugt. Dieser wird in das Dampfnetz der Kolonne zur Abtrennung des Vinylchlorids eingespeist und dient zum Bezeizen von Umlaufverdampfern.

Die Laufzeit des Wärmetauschers beträgt 4 Monate. Während dieser Zeit bildet sich im U-Rohr eine Koksschicht von etwa 0,3 cm Stärke. Der Wärmetauscher wird dann gereinigt. Der durch den indirekten Wärmetausch auf 200°C abgekühlte Gasstrom wird nun in einen Behälter geleitet, in den 1 200 Gewichtsteile 40°C warmes 1,2-Dichlorethan, das aus den Spaltprodukten wiedergewonnen wurde, je 100 Gewichtsteile eingeleitetes Spaltgas eingespritzt wird. Dabei erfolgt eine Abkühlung auf 70°C und eine partielle Kondensation der Spaltprodukte. Durch die Dichlorethanwäsche werden feine Russpartikel aus dem Gasstrom ausgewaschen und aus der flüssigen Dichlorethanphase abfiltriert.

Die Auftrennung der Spaltgase geschieht anschliessend in bekannter Weise durch Abtrennen von Chlorwasserstoff und Vinylchlorid von nicht-umgesetztem 1,2-Dichlorethan und dessen Rückgewinnung.

Es werden 390 kJ/h und kg Spaltgas Wärme vom Temperiermedium aufgenommen, die zur Heizung von Vorrichtungen im Prozess der Auftrennung der Spaltprodukte und des Wiedereinsatzes von rückgewonnenem 1,2-Dichlorethan verwendet werden.

*Beispiel 2*

Es wird nach Schema-Figur 2 verfahren.

Ein Spaltofen wird stündlich mit 9,98 Gewichtsteilen 1,2-Dichlorethan gleicher Zusammensetzung wie in Beispiel 1 beschickt, wobei Spaltendtemperatur und Umsatz dem Beispiel 1 entsprechen. Die Abkühlung der Spaltgase wird in drei Stufen durchgeführt. In der ersten Stufe wird das Spaltgas durch Eindüsen von 2,33 Gewichtsteilen je Stunde flüssigem, bei der Spaltung nicht-umgesetztem auf 40°C abgekühltem 1,2-Dichlorethan von 540°C Eingangstemperatur auf 400°C Ausgangstemperatur abgekühlt. Dabei verdampft das eingespritzte 1,2-Dichlorethan und wird zu einem geringen Anteil in Vinylchlorid und Chlorwasserstoff gespalten. In der zweiten Stufe werden die Spaltgase in einen Einrohr-Wärmetauscher geleitet. Als Temperiermedium wird Mineralöl Marlotherm verwendet, das umgepumpt wird. Die Verweilzeit der Spaltgase im Wärmetauscher beträgt 3,3 s. Am Ausgang des Wärmetauscher-Rohres werden 190°C gemessen. Die durchschnittliche Abkühlgeschwindigkeit der Spaltgase beträgt 63°C/s. Durch indirekten Wärmetausch mit den heissen Spaltgasen wird das Mineralöl auf 180°C erwärmt, wobei 280 kJ/h und kg Spaltgas Wärme aufgenommen werden, die zur Heizung von Vorrichtungen im Prozess der Auftrennung der Spaltprodukte und des Wiedereinsatzes von rückgewonnenem 1,2-Dichlorethan verwendet werden.

In einer dritten Stufe wird das Produkt, das den Wärmetauscher verlässt, wie in Beispiel 1 beschrieben, durch Einspritzen von 40°C warmem 1,2-Dichlorethan auf 70°C abgekühlt und weiter verarbeitet. Nach 6 Monaten hat sich im Innenrohr des Wärmetauschers 2 ein Koksbelag von ca. 3 mm Stärke gebildet. Der Wärmetauscher wird nun zur Verbesserung seines Wirkungsgrades gereinigt.

### Beispiel 3

Es wird nach Schema-Figur 1 verfahren.

Ein Spaltofen wird stündlich mit 45,33 Gewichtsteilen 1,2-Dichlorethan gleicher Zusammensetzung wie in Beispiel 1 beschickt. Der Umsatz beträgt 50%, die Spaltofenausgangstemperatur 500°C. Die Abkühlung der Spaltgase wird zweistufig durchgeführt. In der ersten Stufe erfolgt durch indirekten Wärmetausch in einem Einrohr-Wärmetauscher mit entsalztem Wasser eine Temperaturabsenkung um 370°C von 500°C auf 130°C, wobei stündlich 10,6 Gewichtsteile Dampf mit einem Druck von 0,25 MPa entstehen. Unter dem Systemdruck von 1,7 MPa findet bei einer Temperatur von 130°C eine Teilkondensation von bei der Spaltung nicht-umgesetztem 1,2-Dichlorethan statt. Bei einer Verweilzeit der Spaltgase von 6,2 s und einer durchschnittlichen Abkühlgeschwindigkeit von 60°C/s beträgt die Laufzeit des Wärmetauschers ca. 2 Monate.

Nach Verlassen des Wärmetauschers wird das Produkt von 130°C auf 60°C durch Einspritzen von kaltem 1,2-Dichlorethan abgekühlt und weiter verarbeitet, wie in Beispiel 1 beschrieben.

### Beispiel 4

Es wird nach Schema-Figur 2 verfahren.

Ein Spaltofen wird stündlich mit 45,33 Gewichtsteilen 1,2-Dichlorethan gleicher Zusammensetzung wie in Beispiel 1 beschickt, wobei Spaltendtemperatur und Umsatz dem Beispiel 3 entsprechen. Die Abkühlung der Spaltgase wird wie in Beispiel 2 dreistufig durchgeführt.

In der ersten Stufe werden die Spaltgase durch Einspritzen von stündlich 10,46 Gewichtsteilen flüssigem, bei der Spaltung nicht-umgesetztem, 40°C warmem 1,2-Dichlorethan auf 370°C abgekühlt.

In der zweiten Stufe erfolgt durch indirekten Wärmetausch die Abkühlung auf 130°C, wobei 10 Gewichtsteile Dampf/Stunde entstehen. Bei einer Verweilzeit der Spaltgase im Wärmetauscher von 6 s und einer durchschnittlichen Abkühlgeschwindigkeit von 40°C/s beträgt die Laufzeit des Wärmetauschers etwa 4 Monate. Um den Spaltvorgang während der Kühlerreinigung nicht unterbrechen zu müssen, wird ein in Reserve stehender zweiter Wärmetauscher in Betrieb genommen.

Nach Verlassen des Wärmetauschers wird das Produkt von 130°C auf 60°C durch Einspritzen von kaltem 1,2-Dichlorethan abgekühlt und weiterverarbeitet, wie in Beispiel 1 beschrieben.

### Vergleichsversuch A

Es wird nach Schema-Figur 1 verfahren.

Der Versuchsablauf erfolgt in analoger Weise wie in Beispiel 1 mit der Änderung, dass die durchschnittliche Abkühlgeschwindigkeit der heissen Spaltgase von 540°C auf 200°C nur 20°C/s beträgt. Bereits nach wenigen Tagen Laufzeit steigt der Druckabfall im Kühler von 0,2 auf 0,5 MPa. Das Wärmetauscherrohr hat nach 2 Wochen Laufzeit einen Koksbelag von 5 mm Stärke.

### Vergleichsversuch B

Es wird nach Schema-Figur 2 verfahren.

Der Versuchsablauf erfolgt in analoger Weise wie in Beispiel 4 mit der Änderung, dass die durchschnittliche Abkühlgeschwindigkeit der heissen Spaltgase von 370°C auf 130°C nur 15°C/s beträgt. Hier stellt sich ein ähnlicher Verlauf wie in Vergleichsversuch A ein. Es erfolgt eine sehr rasche Koksbildung im Wärmetauscher. Die Laufzeit des Wärmetauschers beträgt weniger als 4 Wochen.

### Beispiel 5

Es wird nach Schema-Figur 3 verfahren.

Ein Spaltofen wird stündlich mit 9,98 Gewichtsteilen 1,2-Dichlorethan der in Beispiel 1 beschriebenen Zusammensetzung beschickt. Bei einem Druck von 2,1 MPa werden stündlich 5,46 Gewichtsteile des 1,2-Dichlorethans gespalten. Der Umsatz beträgt 55%.

Die den Spaltofen verlassenden 540°C heissen Gase werden durch einen Einröhren-Wärmetauscher 2 mit einem einseitig angeflanschten U-förmigen Rohr geleitet. Als Temperiermedium wird vollentsalztes Wasser 3 verwendet. Die Verweilzeit der Spaltgase im Wärmetauscher beträgt 4 s. Am Ausgang des U-Rohres werden 220°C gemessen. Die durchschnittliche Abkühlgeschwindigkeit der Spaltgase beträgt 85°C/s bei einem Druckverlust der Spaltgase im Wärmetauscher von 0,2 MPa.

Durch indirekten Wärmetausch mit den heissen Spaltgasen werden stündlich 1,4 Gewichtsteile Wasserdampf 4 mit einem Druck von 0,8 MPa erzeugt. Dieser wird in das Dampfnetz der Kolonne zur Abtrennung des Vinylchlorids eingespeist und dient zum Beheizen von Umlaufverdampfern. Die Laufzeit des Wärmetauschers beträgt 6 Monate.

Der durch den indirekten Wärmetausch auf 220°C abgekühlte Gasstrom wird nun in einem zweiten Wärmetauscher 24 der gleichen U-förmigen Bauart, jedoch um 40% verminderten Rohrquerschnittes weiter auf 140°C abgekühlt. Die durchschnittliche Abkühlgeschwindigkeit beträgt 85°C/s bei einem Druckverlust der Spaltgase im Wärmetauscher von 0,1 MPa. Es werden stündlich 0,6 t Wasserdampf bei einem Druck von 0,2 MPa gewonnen. Die rückgewonnene Energie dient vorwiegend zur Destillation von 1,2-Dichlorethan.

Zum Austrag des Russes aus dem zweiten Wärmetauscher wird das Spaltgas vor Eintritt in den zweiten Wärmetauscher diskontinuierlich alle 100 Stunden 5 Minuten lang 2,7 kg/min vorwiegend 1,2-Dichlorethan enthaltender Flüssigkeit aus Apparat 6 je kg/min Spaltgas über Leitung 28 zugeführt. Die Temperatur der zugeführten Flüssigkeit beträgt 60°C.

Der Wärmetauscher erreicht eine Laufzeit von 6 Monaten. Während dieser Zeit bildet sich im U-Rohr eine Koksschicht von etwa 4 mm Stärke. Zur weiteren Abkühlung und Auftrennung des Gasstromes wird analog Beispiel 1 verfahren.

Der auf 140°C abgekühlte Gasstrom wird nun in einen Behälter 6 geleitet, in den 70 Gewichtsteile 40°C warmes 1,2-Dichlorethan 5, das aus den Spaltprodukten wiedergewonnen wurde, je 10 Gewichtsteile eingeleitetes Spaltgas eingespritzt wird. Dabei erfolgt eine Abkühlung auf 60°C und weitere Kondensation der Spaltprodukte. Durch die Dichlorethan-Wäsche werden feine Russpartikel aus dem Gasstrom ausgewaschen und aus der flüssigen Dichlorethanphase in der Vorrichtung 27 ausfiltriert. Die Auftrennung der Spaltgase geschieht anschliessend in bekannter Weise durch Abtrennen von Chlorwasserstoff aus dem Vinylchlorid und nicht-umgesetztem Dichlorethan in der sogenannten HCl-Kolonne 13.

Die Kolonne wird mit drei Produktströmen gespeist, einem Flüssigstrom, der vorwiegend aus 1,2-Dichlorethan besteht über Leitung 8, einem weiteren Flüssigstrom über Leitung 11, der vorwiegend aus Vinylchlorid besteht und einem gasförmigen, vorwiegend chlorwasserstoffhaltigen Strom über Leitung 12. Zur Auftrennung der Gasphase aus Behälter 6 in eine weitere Flüssig- und Gasphase wird der Gasstrom durch indirekten Wärmetausch im Apparat 10 auf ca. 20°C abgekühlt.

## Patentansprüche

1. Verfahren zur Rückgewinnung von Pyrolyseenergie bei der Herstellung von Vinylchlorid durch thermische Spaltung von 1,2-Dichlorethan, wobei das Spaltgasgemisch, das den Reaktionsofen verlässt, indirekt mit einem flüssigen oder gasförmigen Temperiermedium gekühlt und anschliessend destillativ aufgetrennt wird, dadurch gekennzeichnet, dass die gesamte Kühlung in mindestens 2 Stufen durchgeführt wird, wobei das Spaltgasgemisch mit einer durchschnittlichen Abkühlgeschwindigkeit von mindestens pro Sekunde 1/10 der Temperatur in °C, mit der dieses Gemisch in die Zone der indirekten Kühlung eintritt, bis zum Erreichen einer Temperatur von 220 bis 120°C gekühlt, die dabei an das Temperiermedium abgegebene Wärme zur Heizung von Vorrichtungen verwendet und das Spaltgasgemisch gegebenenfalls zusammen mit dem daraus gebildeten flüssigen Kondensat in mindestens einer anschliessenden Stufe direkt oder indirekt weiter abgekühlt wird.

2. Verfahren nach Anspruch 1, dadurch gekennzeichnet, dass das Spaltgasgemisch, welches den Reaktionsofen mit einer Temperatur von ca. 560 bis 480°C verlässt, in der ersten Stufe durch Inkontaktbringen mit einem flüssigen, mindestens 50 Gew.-% 1,2-Dichlorethan enthaltenden Produkt direkt sowie nachfolgend mit einem flüssigen oder gasförmigen Temperiermedium indirekt gekühlt wird, wobei die indirekte Kühlung erst einsetzt, wenn das Spaltgasgemisch eine Temperatur von etwa 430 bis 350°C erreicht hat, und bis zu einer Temperatur von 220 bis 120°C weitergeführt wird, wobei ferner die an das Temperiermedium abgegebene Wärme zur Heizung von Vorrichtungen verwendet und das Spaltgasgemisch gegebenenfalls zusammen mit dem daraus gebildeten Kondensat in mindestens einer anschliessenden Stufe direkt oder indirekt weiter abgekühlt wird.

3. Verfahren nach Ansprüchen 1 und 2, dadurch gekennzeichnet, dass das Spaltgasgemisch mit einer durchschnittlichen Abkühlgeschwindigkeit von pro Sekunde 1/4 bis 1/9 der Temperatur in °C, mit der dieses Gemisch in die Zone der indirekten Kühlung eintritt, bis zum Erreichen einer Temperatur von 220 bis 120°C gekühlt wird.

4. Verfahren nach Ansprüchen 2 bis 3, dadurch gekennzeichnet, dass die indirekte Kühlung bei einer Temperatur von 400 bis 370°C einsetzt.

5. Verfahren nach Anspruch 1, dadurch gekennzeichnet, dass das Spaltgasgemisch in einer ersten Stufe durch indirekte Kühlung von der Spaltofen-Ausgangstemperatur auf etwa 220°C, dann in einer zweiten Stufe unter absatzweiser Zuführung einer im wesentlichen 1,2-Dichlorethan enthaltenden Flüssigkeit durch teilweise direkte, teilweise indirekte Kühlung auf etwa 140°C und anschliessend in mindestens einer weiteren Stufe durch direkte oder indirekte Kühlung auf die Eingangstemperatur zu der Kolonne, in der Chlorwasserstoff aus den Produkten der thermischen Spaltung von 1,2-Dichlorethan abgetrennt wird, gekühlt wird.

6. Verfahren nach Anspruch 5, dadurch gekennzeichnet, dass die im wesentlichen 1,2-Dichlorethan enthaltende Flüssigkeit in Abständen von 20 bis etwa 500 Stunden während einer Zeit von je 1 bis etwa 30 Minuten in einer Menge von 1,5 bis 5,0 kg/min der genannten Flüssigkeit je kg/min Spaltgas in die zweite Stufe der Kühlung eingegeben wird.

7. Verfahren nach Ansprüchen 5 und 6, dadurch gekennzeichnet, dass die im wesentlichen 1,2-Dichlorethan enthaltende Flüssigkeit vor der Zugabe zur zweiten Stufe der Kühlung eine Temperatur von 20 bis 70°C aufweist.

8. Verfahren nach Ansprüchen 1 bis 7, dadurch gekennzeichnet, dass in der ersten Stufe zur indirekten Kühlung ein bei 100°C flüssiges Temperiermedium eingesetzt wird, dessen Dampfdruck bei 220°C 2,5 MPa nicht übersteigt.

9. Verfahren nach Ansprüchen 1 bis 8, dadurch gekennzeichnet, dass in der ersten Stufe zur indirekten Kühlung ein Temperiermedium eingesetzt wird, dessen Siedepunkt bei einem Druck von 98,1 kPa zwischen 70 und 110°C liegt.

10. Verfahren nach Ansprüchen 1 bis 9, dadurch gekennzeichnet, dass das Temperiermedium 1,2-Dichlorethan ist.

## Claims

1. A process for the manufacture of vinyl chloride by thermal cracking of 1,2-dichloroethane, with the reaction gas leaving the reaction furnace being cooled indirectly by a liquid or gaseous coolant for subsequent splitting by distillation, said process incorporating the improvement which comprises performing the complete cooling in at least 2 stages, the reaction gas mixture being cooled to a temperature level of 220 to 120 °C at an average cooling rate per second of at least 1/10 of the temperature in terms of °C at which this mixture penetrates into the zone of indirect cooling, the heat transferred to the coolant being utilized for heating any process equipment and the reaction gas mixture together with any condensate resulting from this mixture being further cooled directly or indirectly in at least one downstream stage.

2. A process according to claim 1, incorporating the improvement which comprises the reaction gas mixture leaving the reaction furnace at a temperature of 560 to 480°C being cooled directly in the first stage by contacting it with a liquid product containing at least 50% by weight of 1,2-dichloroethane and being subsequently cooled indirectly with a liquid or gaseous coolant, the indirect cooling being initiated only when the reaction gas mixture has reached a temperature level of about 430 to 350°C and being continued to a temperature level of 220 to 120°C, the heat transferred to the coolant being utilized for heating any process equipment and the reaction gas mixture together with any condensate resulting from this mixture being further cooled directly or indirectly in at least one downstream stage.

3. A process according to claims 1 and 2, incorporating the improvement which comprises cooling the reaction gas mixture to a temperature level of 220 to 120°C at an average cooling rate per second of 1/4 to 1/9 of the temperature in terms of °C at which this mixture penetrates into the zone of indirect cooling.

4. A process according to claims 2 to 3, incorporating the improvement which comprises initiating the indirect cooling at a temperature of 400 to 370°C.

5. A process according to claim 1, incorporating the improvement which comprises cooling the reaction gas mixture in a first stage by indirect cooling from the reaction furnace outlet temperature to about 220°C, lowering its temperature to about 140°C by partly direct, partly indirect cooling in a second stage while a liquid substantially consisting of 1,2-dichloroethane is added intermittently, and subsequently cooling it directly or indirectly in at least one further stage to the inlet temperature of the column in which the hydrogen chloride is separated from the products of thermal cracking of 1,2-dichloroethane.

6. A process according to claim 5, incorporating the improvement which comprises admitting the liquid substantially consisting of 1,2-dichloroethane to the second cooling stage at intervals of 20 to about 500 hours for a period of 1 to about 30 minutes and at a rate of 1.5 to 5.0 kg/min per kg/min of reaction gas.

7. A process according to claims 5 and 6, incorporating improvement which comprises the liquid substantially consisting of 1,2-dichloroethane having a temperature of 20 to 70°C before being admitted to the second cooling stage.

8. A process according to claims 1 to 7, incorporating the improvement which comprises using in the first stage for indirect cooling a coolant which is liquid at 100°C and whose vapour pressure does not exceed 2.5 MPa at 220 °C.

9. A process according to claims 1 to 8, incorporating the improvement which comprises using for indirect cooling in the first stage a coolant whose boiling point is situated between 70 and 110°C at a pressure of 98.1 kPa.

10. A process according to claims 1 to 9, incorporating the improvement which comprises the coolant being 1,2-dichloroethane.

**Revendications**

1. Procécé de fabrication de chlorure de vinyle par le craquage thermique de dichloréthane 1-2, le mélange de gaz de réaction qui sort du four de réaction étant refoidi indirectement par un fluide réfrigérant liquide ou gazeux et ensuite fractionné par distillation, caractérisé en ce que l'ensemble du processus de refroidissement a lieu dans deux étages au moins, le mélange de gaz de réaction étant refroidi à une vitesse moyenne de refroidissement, par seconde, de 1/10 de la température en °C à laquelle ledit mélange est admis dans la zone de refroidissement indirect, jusqu'à ce qu'il ait atteint une température de 220 à 120°C, la chaleur transmise au fluide réfrigérant étant utilisée pour le chauffage d'appareils et le mélange de gaz de réaction étant encore refroidi, directement ou indirectement, dans au moins un autre étage installé en aval, le cas échéant, avec le condensat liquide qui s'est formé.

2. Procédé selon la revendication 1, caractérisé en ce que le mélange de gaz de réaction qui sort du four de réaction à la température de 560 à 480°C environ est refroidi directement dans le premier étage par le contact avec un produit liquide contenant au moins 50% en poids de dichloréthane 1-2, puis indirectement par un fluide réfrigérant liquide ou gazeux, le refroidissement indirect ne commençant que lorsque le mélange de gaz de réaction a atteint une température approximative de 430 à 350°C, et il est poursuivi jusqu'à ce qu'une température de 220 à 120°C soit obtenue, étant entendu que la chaleur transmise au fluide réfrigérant est de plus utilisée pour le chauffage d'appareils et que le mélange de gaz de réaction est encore refroidi directement ou indirectement dans un autre étage au moins installé en aval, le cas échéant, avec le condensat qui s'est formé.

3. Procédé selon les revendications 1 et 2, caractérisé en ce que le mélange de gaz de réaction est refroidi à une vitesse moyenne de refroidissement, par seconde, de 1/4 à 1/9 de la température en °C à laquelle ledit mélange est admis dans la zone de refroidissement indirect, jusqu'à ce que la température de 220 à 120°C soit atteinte.

4. Procédé selon l'une quelconque des revendications 2 à 3, caractérisé en ce que le refroidissement indirect ne commence qu'à la température de 400 à 370°C.

5. Procédé selon la revendication 1, caractérisé en ce que le mélange de gaz de réaction est refroidi dans un premier étage par refroidissement indirect de la température de sortie du four de réaction à la température de 220°C environ, puis dans un deuxième étage par refroidissement direct ou indirect à la température de 140°C dû à l'admission intermittente d'un liquide composé surtout de dichloréthane 1-2 et, enfin, dans au moins un autre étage par refroidissement direct ou indirect à la température d'admission dans la colonne où le gaz hydrochlorique est séparé des produits provenant du craquage thermique du dichloréthane 1-2.

6. Procédé selon la revendication 5, caractérisé en ce que le liquide composé principalement de dichloréthane 1-2 est admis dans le deuxième étage de refroidissement, à un intervalle de 20 à 500 heures environ et pendant une durée de 1 à 30 minutes environ, avec une proportion de 1,5 à 5,0 kg/min dudit liquide par kg/min de gaz de réaction.

7. Procédé selon les revendications 5 et 6, caractérisé en ce que le liquide composé surtout de dichloréthane 1-2 a une température de 20 à 70°C avant d'être ajouté dans le deuxième étage de refroidissement.

8. Procédé selon l'une quelconque des revendications 1 à 7, caractérisé en ce que, pour le refroidissement indirect dans le premier étage, un fluide réfrigérant, liquide, à une température de 100°C est mis en œuvre, sa pression de vapeur à 220°C n'excédant pas 2,5 MPa.

9. Procédé selon l'une quelconque des revendications 1 à 8, caractérisé en ce que pour le refroidissement indirect dans le premier étage, un fluide réfrigérant est mis en œuvre dont le point d'ébullition est situé entre 70 et 110°C à une pression de 98,1 kPa.

10. Procédé selon l'une quelconque des revendications 1 à 9, caractérisé en ce que le fluide réfrigérant mis en œuvre est du dichloréthane 1-2.

Fig. 1

Fig. 2

Fig. 3